# EUROPEAN PATENT SPECIFICATION

(11) **EP 1 556 703 B1**
(45) Date of publication and mention of the grant of the patent: **22.10.2008**
(21) Application number: 03776874.4
(22) Date of filing: 06.10.2003
(51) Int. Cl.: G01N 33/68

(54) **INHIBITORS OF SRC KINASE FOR USE IN ALZHEIMER'S DISEASE**
SRC KINASE INHIBITOREN ZUR BEHANDLUNG VON ALZHEIMER KRANKHEIT
INHIBITEURS DE LA SRC KINASE DESTINES A ETRE UTILISES DANS LA MALADIE D'ALZHEIMER

(30) Priority: 22.10.2002 EP 02292608
(43) Date of publication of application: 27.07.2005
(73) Proprietor: Aventis Pharma S.A., 92160 Antony (FR)
(72) Inventor: MERCKEN, Luc, F-94100 Saint Maur (FR); ZAMBRANO, Nicola, I-80020 Casavatore (NA) (IT); RUSSO, Tommaso, I-80131 Napoli (IT)
(74) Representative: Bouvet, Philippe
(86) International application number: PCT/EP2003/012063
(87) International publication number: WO 2004/038422

(56) References cited:
- WO-A-01/45751
- WILLIAMSON R ET AL: "RAPID TYROSINE PHOSPHORYLATION OF NEURONAL PROTEINS INCLUDING TAU AND FOCAL ADHESION KINASE IN RESPONSE TO AMYLOID-BETA PEPTIDE EXPOSURE: INVOLVEMENT OF SRC FAMILY PROTEIN KINASES" JOURNAL OF NEUROSCIENCE, NEW YORK, NY, US, vol. 22, no. 1, 1 January 2002 (2002-01-01), pages 10-20, XP009002922 ISSN: 0270-6474 cited in the application
- SLACK B E ET AL: "A ROLE FOR SRC TYROSINE KINASE IN THE STIMULATION OF AMYLOID PRECURSOR PROTEIN RELEASE BY ACTIVATION OF MUSCARINIC M3 ACETYLCHOLINE RECEPTORS" SOCIETY FOR NEUROSCIENCE ABSTRACTS, SOCIETY FOR NEUROSCIENCE, US, vol. 24, no. 1/2, 7 November 1998 (1998-11-07), page 208,AB8412 XP009002921 ISSN: 0190-5295 cited in the application
- KIHARA T ET AL: "ALPHA7 NICOTINIC RECEPTOR TRANSDUCES SIGNALS TO PHOSPHATIDYLINOSITOL 3-KINASE TO BLOCK A BETA-AMYLOID-INDUCED NEUROTOXICITY" JOURNAL OF BIOLOGICAL CHEMISTRY, AMERICAN SOCIETY OF BIOLOGICAL CHEMISTS, BALTIMORE, MD, US, vol. 276, no. 17, 27 April 2001 (2001-04-27), pages 13541-13546, XP001146597 ISSN: 0021-9258
- GIANNI DAVIDE ET AL: "Platelet-derived growth factor induces the beta-gamma-secretase-medi ated cleavage of Alzheimer's amyloid precursor protein through a Src-Rac-dependent pathway." JOURNAL OF BIOLOGICAL CHEMISTRY, vol. 278, no. 11, 14 March 2003 (2003-03-14), pages 9290-9297, XP002282954 ISSN: 0021-9258
- HANKE J ET AL: "Discovery of a novel, potent, and Src family-selective tyrosine kinase inhibitor" JOURNAL OF BIOLOGICAL CHEMISTRY, vol. 271, no. 2, 12 January 1996 (1996-01-12), pages 695-701, XP002051826 ISSN: 0021-9258

## Description

The present invention relates to the identification of inhibitors of Src kinase, assays and methods useful for such identification and the use of Src inhibitor PP2 for the preparation of pharmaceuticals for the treatment of Alzheimer's disease.

Alzheimer's disease is a neurodegenerative disorder of the brain, which is accompanied at the cellular level by a massive loss of neurons in the limbic system and in the cerebral cortex. In the brain areas affected, protein deposits, so-called plaques, can be detected at the molecular level, which are an essential characteristic of Alzheimer's disease. The protein occurring most frequently in these plaques is a peptide of 40 to 42 amino acids in size, which is designated as Aß-peptide. This peptide is a cleavage product of a larger protein of 695 to 770 amino acids, the so-called Amyloid Precursor Protein (APP).

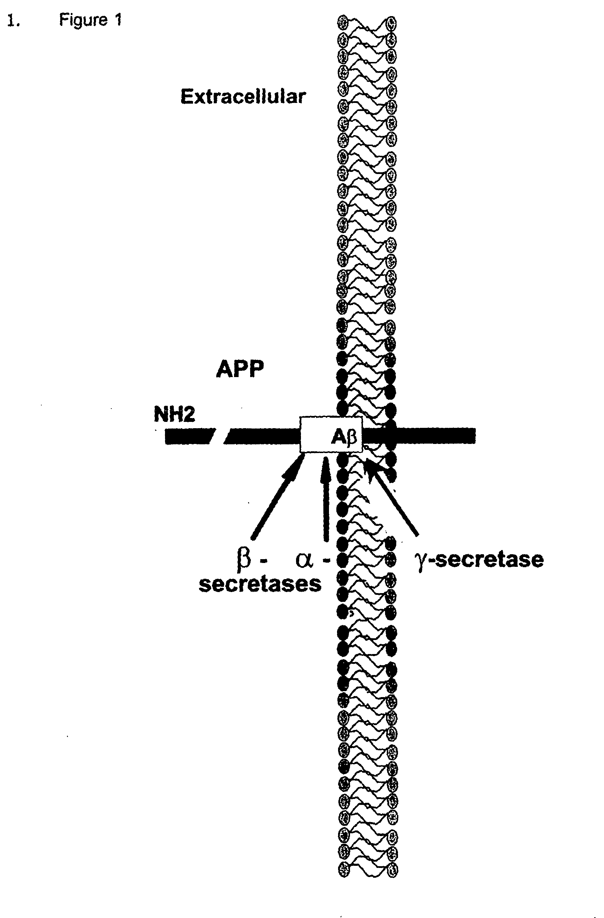

APP is an integral transmembrane protein, which firstly traverses the lipid bilayer. By far the largest part of the protein is extracellular, while the shorter C-terminal domain is directed into the cytosol (Figure 1). APP is the substrate of three different proteases: α-secretase, β-secretase and γ-secretase. Within APP, about two thirds of the Aß-peptide originates from the extracellular domain and about one third from the transmembrane domain.

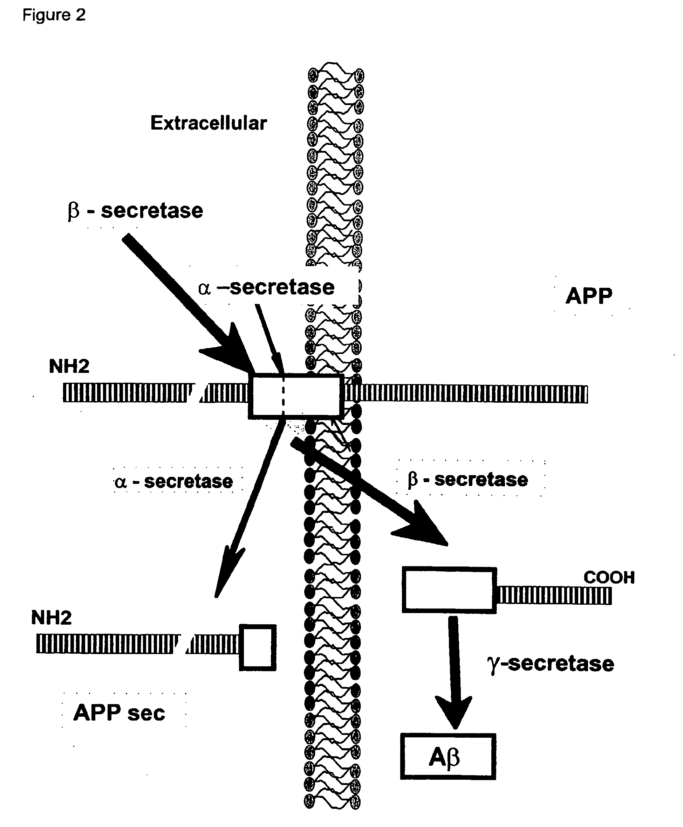

Beside the membrane-linked APP, a secreted form of APP can be detected which consists of the large ectodomain of APP and is designated as APP_{sec} ("secreted APP"). APP_{sec} is formed from APP by proteolytic cleavage, which is effected by the α-secretase (Figure 2). This proteolytic cleavage takes place within the amino acid sequence of the Aß-peptide (after amino acid residue 16 of the Aß-peptide). Proteolysis of APP by the α-secretase thus excludes the formation of the Aβ-peptide.

The Aβ-peptide can thus only be formed from APP in an alternative processing route. It is postulated that two proteases are involved in this processing, one protease, which is designated as ß-secretase, cleaving at the N-terminus of the Aß-peptide in the APP and the second protease, which is designated as γ-secretase, releasing the C-terminus of the Aβ-peptide (kang, J. et al., (1987) Nature, 325, 733 (Figure 2).

There are many indications that the Aß-peptide is a crucial factor in the occurrence of Alzheimer's disease. Inter alia, neurotoxicity of Aβ-fibrils in cell culture is postulated (Yankner, B.A. et al., (1990) Proc Natl Acad Sci USA, 87, 9020). In patients with Down's syndrome, in which APP occurs in an additional copy, the neuropathology characteristic of Alzheimer's disease also occurs even at an age of 30 years. Here, it is assumed that the overexpression of APP follows an increased conversion into the Aß-peptide (Rumble, B. et al., (1989), N. Engl. J. Med., 320, 1446).

Furthermore the strongest indication of the central role of the Aß-peptide in Alzheimer's disease are the familial forms of the disease. Here, mutations are found in the APP gene around the area of the secretase cleavage sites or in two further AD-associated genes (presenilins), which in cell culture lead to a significant increase in Aβ production (Scheuner, D. et al., (1996), Nature Medicine, 2, 864).

There are a number of indications that APP is first cleaved by the β-secretase, to serve thereafter as a substrate for γ-secretase (Maruyama, K. Y. et al., (1994) Biochem. Biophys Res Commun, 202, 1517; Estus, S. et al., (1992), Science, 255, 726 ). The γ-secretase therefore has a crucial role in the formation of the Aβ-peptide. A demonstration of the activity of the γ-secretase which is customarily used is the detection of the Aß-peptide, which, however, frequently turns out to be difficult.

An important reason for this is that only a small part of APP is converted into the Aβ-peptide (Simons M, et al., Neurosci (1996) 1;16(3):899-908). Moreover, the Aβ-peptide is a small breakage fragment of about 4 kDa and, on account of its hydrophobic character, has a great tendency to self-aggregation so that it easily precipitates under physiological conditions (Hilbich, C. et al., (1991) J. Mol. Biol., 218, 149).

The short, 47 amino acid-long C-terminal tail of APP is exposed to the cytosol and is the site of interaction for molecular adaptors containing PTB domains, named Fe65, X11, m-dab and Jip1. Since the binding of these proteins is dependent on the YENPTY sequence on APP, it is expected that their interactions are not simultaneous (T. Russo et al. (1998) FEBS Lett. 434, 1-7; N. Zambrano et al. (1997) J. Biol. Chem. 272, 6399-6405). Indeed, it has been shown, in cultured cells, that Fe65 and X11 proteins have opposite effects on the proteolytic processing of APP (Sabo S. et al. (1999) J. Biol. Chem. 274, 7952-7957; Borg J.P. et al. (1998) J. Biol. Chem. 273, 14761-14766). A possible explanation for these findings may reside in the recruitment, by either Fe65 or X11, of APP in different macromolecular complexes, depending on the interaction of different sets of proteins with the other protein-protein interaction domains of the two adaptors.

The APP cytodomain contains various serine and threonine residues, which are phosphorylated *in vitro* by different kinase activities. Thr668 is a main site of phosphorylation *in vivo,* being phosphorylated by neuronal cyclin-dependent kinases, Cdk5 in neurons (lijima, K. et al. (2000) J. Neurochem. 75, 1085-1091), cdc2 kinase in dividing cells (Suzuki, T. et al. (1994) EMBO J. 13, 1114-1122 ; Oishi, M. et al. (1997) Mol. Med. 3, 111-123), and glycogen synthase kinase 3b (GSK3b) and stress-activated protein kinase 1b (SAP kinase1b) *in vitro* (Aplin, E. E. et al. (1996) J. Neurochem. 67, 699-707 ; Standen, C. L. et al. (2001) J. Neurochem. 76, 316-320). It has been shown that phosphorylation of APP regulates neurite extension in differentiating PC12 cells (Ando, K. et al. (1999) J. Neurosci. 19, 4421-4427), while, at the molecular level, APP phosphorylation may regulate the binding to the PTB domain-containing adaptors and its processing (Ando K. et al. (2001) J Biol. Chem., 276, 40353-40361).

Previously it was demonstrated that APP is phosphorylated at tyrosine 682 in cells expressing an active form of the Abl non receptor tyrosine kinase (Zambrano N. et al. (2001) J Biol. Chem. 276, 19787-92); active Abl is recruited in proximity to APP by Fe65, which may bind simultaneously these two proteins through its WW domain (Abl) and PTB2 domain (APP). Phosphorylation of Tyr682 of APP generates a docking site for the SH2 domain of Abl and, in fact stable complexes between APP and Abl are formed.
In order to test the hypothesis that tyrosine phosphorylation regulates pp60c-src biological activity, Kmiecik and Shalloway (1987) Cell 49, 65-73 described the construction and study of pp60c-src mutants in which Tyr 527 and Tyr 416 were separately or coordinately altered to phenylalanine. Tyr---- Phe 527 mutation strongly activated pp60c-src transforming and kinase activities, whereas the additional introduction of a Tyr----Phe 416 mutation suppressed these activities. Tyr---Phe 416 mutation of normal pp60c-src eliminated its partial transforming activity, which suggests that transient or otherwise restricted phosphorylation of Tyr 416 is important for pp60c-src function even though stable phosphorylation is not observed in vivo. Normally, pp60c-src is phosphorylated in vivo at tyrosine 527, a residue not present in pp60v-src (its transforming homolog), and not at tyrosine 416, its site of in vitro autophosphorylation.

Williamson et al. (2002) J. Neurochem. 22, 10-20) described the rapid phosphorylation of neuronal proteins including Tau and Focal adhesion kinase in response to amyloid-β peptide exposure and an involvement of Src family protein kinases.

Slack, B. E. and Berse; B. (1998) described in Society for Neuroscience Abstracts, Vol. 24, No. 1-2, pp. 208 (Conference/Meeting Information: 28th Annual Meeting of the Society for Neuroscience, Part 1, Los Angeles, California, USA, Nov. 7-12, 1998 Society for Neuroscience, ISSN: 0190-5295) a role for tyrosine kinases in the stimulation of APP release by action of muscarinic m3 acetylcholine receptors. They described a role for Src tyrosine kinase in the regulation of APP_{sec} release by muscarinic receptors. They demonstrated that an increase in the active form of Src leads to a decrease in secAPP.

The present invention provides methods of identifying therapeutic agents for the treatment of Alzheimer's disease.

One embodiment of the invention provides methods for identifying inhibitors of Src activity, whereby the methods comprise the steps
a) providing a Src protein (i.e. a Src encoding DNA under control of an expression element) and
b) determining if a compound inhibits the activity of Src.

The Src protein could be any mammalian Src protein, preferably human Src (SEQ ID NO. 1 (isoform 1) and SEQ ID NO. 2 (isoform 2)) or rodent Src (SEQ ID NO. 3). The protein could be obtained by expressing human Src or rodent Src cDNA, by using e. g. sequences SEQ ID NO. 4 or SEQ ID NO. 5. In addition, one of the sequences deposited under Genbank Accession No. M17031 or BC011566 can be used.

For the method preferably a mammalian cell or cell line is used in which Src is expressed, either naturally or because the cell / cell line is genetically engineered. In a particular embodiment primary cultures of neurones are used.

Another embodiment of the invention relates to a method of identifying compounds, which inhibit Src expression. The method comprises the steps
a) providing a sequence which regulates Src expression (i.e. a Src promotor sequence) and
b) determining if a compound inhibits the expression of Src protein.

The sequence which regulates Src expression is preferably linked to a reporter gene or a reporter gene construct. Such reporter gene can easily be used to determine, if a compound inhibits the expression of Src. Another possibility is the use of the Src gene. The region of chromosome 20 including the Src gene is deposited under Genbank Accession no AL133293 .

An house-keeping promoter, which can be used as a sequence which regulates Src expression, has been described, as well as an alternative promoter regulated by the Hepatic Nuclear Factor-1a (Bonham et al. (2000) J. Biol. Chem. 275, 37604) (Genbank accession number AF272982).

Another embodiment of the invention refers to the use of PP2 (4-amino-5-(4-chlorophenyl)-7-(t-butyl)pyrazolo[3,4-d]pyrimidine) for the preparation of a pharmaceutical for the treatment of Alzheimer's disease.

The new results demonstrate, that APP is phosphorylated not only by Abl. In fact Src kinase is also responsible for APP tyrosine phosphorylation. It is expected that tyrosine phosphorylation of APP alters the binding properties of its cytodomain, and then the processing of APP could be consequently modified. If so, this will prove useful the use of currently available tyrosine kinase inhibitors, and to design new ones for pharmacological modulation of the release of APP processing products in cellular and animal models of Alzheimer's disease.
Taken together, the results from the following examples suggest that exogenous Src activity is responsible for activation of a pathway resulting in increased Aβ secretion. This also suggests that PP2 could be used as a pharmacological agent able to reduce Aβ secretion in cellular and animal systems resembling AD status.

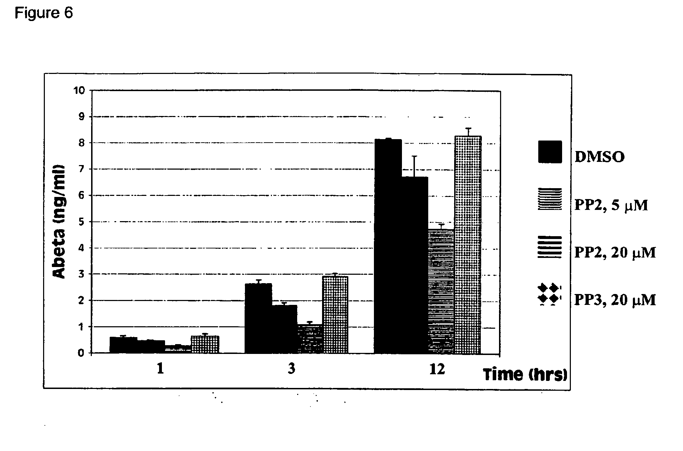

Therefore, a line of CHO cells stably overexpressing APP751 form was used, which secretes discrete levels of Aβ. If secretion involves endogenous Src activity, PP2 should be able to inhibit production of Aβ also in this cellular system. This is indeed the case since, as shown in Figure 6, PP2 reduces in a dose-dependent manner the amount of Aβ at the three time points tested (1, 3 and 12 hours), while PP3, used at the highest concentration, has no effect.
These experiments clearly outline the relevance of Src activation in Aβ secretion and constitute the basis to try pharmacological applications for tyrosine kinase inhibitors in the treatment of AD. Screening of such inhibitors and their optimization will lead to the development of potential, innovative therapeutic agents for treatment of Alzheimer's disease.

### Examples

### Example 1

### Cell culture, transfections and treatments

Human embryonic kidney cells (HEK293) were cultured in Dulbecco's modified minimal medium supplemented with 10% fetal calf serum at 37°C in a 5% CO₂ atmosphere. For transfection, cells were grown for 16 hours in antibiotic-free medium, and transfected with Lipofectamine 2000 (Invitrogen) according to manufacturer's instructions. The total amount of DNA was maintained constant by addition of empty vector DNA. 48 hours after transfection, culture medium were recovered, while cells were harvested in ice-cold phosphate buffer saline (PBS) and gently lysed in lysis buffer (50 mM Tris/HCl, pH 7.5, 150 mM NaCl, 0.5% Triton X-100, 10% glycerol, 50 mM NaF, 1mM Na vanadate), in the presence of a protease inhibitor cocktail (Complete, EDTA-free, Roche). The extracts were clarified by centrifugation at 16,000 x g at 4 °C, and the protein concentration determined by the Bio-Rad protein assay according to manufacturer's instructions. For metabolic labelling, 36 hours after transfection, cells were incubated for 30 minutes in medium without methionine and cysteine, then the medium was replaced with fresh medium containing 80 µCi/ml of ³⁵S-methionine/cysteine mixture (Promix, 1000 Ci/mmol, Amersham Pharmacia Biotech). Pulse was for 30 minutes, then the radioactive medium was replaced with complete medium for 90 minutes. CHO cells expressing wild-type APP751 have been described (N. Zambrano et al. (1997) J. Biol. Chem. 272, 6399-6405).
Immuno-precipitations were performed either on culture medium, or on cellular lysates with 1 µg/sample of the 6E10 monoclonal antibody (Signet).
Radioactive samples were resolved on 10% SDS-PAGE and quantitated with a Storm 840 phosphorimager (Amersham Pharmacia Biotech).
PP2 and PP3 (Hanke, J.H. et al. (1996) J. Biol. Chem. 271, 695-701) were purchased from Calbiochem and dissolved in DMSO; treatments were performed on transfected cells for 48 hours with vehicle alone, or with 1, 5 and 20 µM PP2 or PP3. Concentration of DMSO was the same in all samples. Treatments on CHO/APP751 cells was made with DMSO, or PP2 (5 and 20 µM), or PP3 (20 µM).

### Example 2

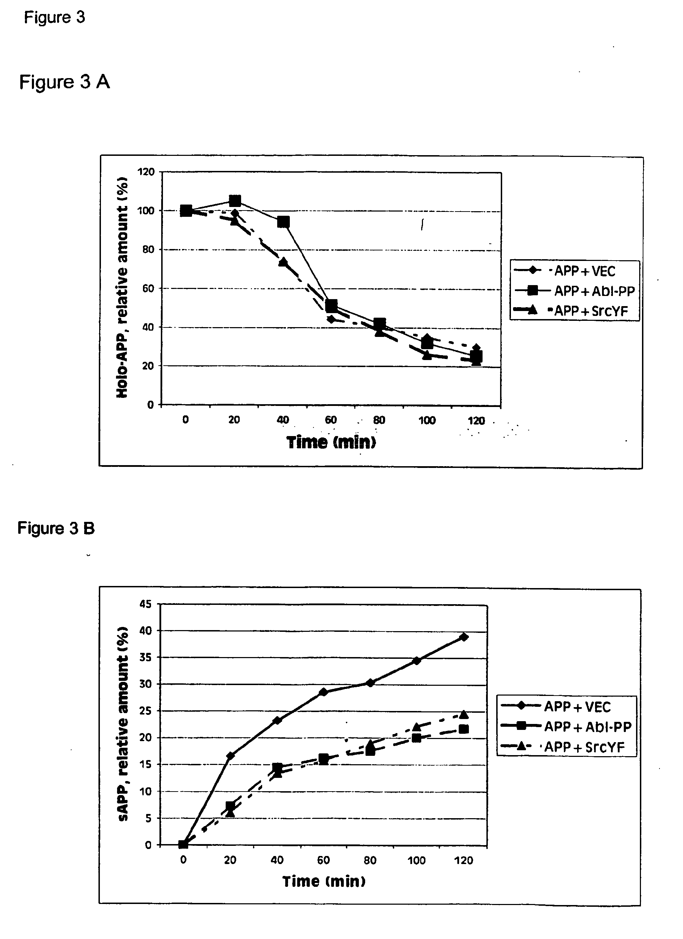

HEK293 cells were transfected with APP695 expression vectors. This cellular system was used because these cells are transfected to high efficiency, and because they express the whole set of processing activities required for APP maturation. In fact, upon transfection of human APP695 expression Vectors, both α-secretase product (APP_{sec}) and β- γ-secretase products (Aβ) accumulate in the culture medium. HEK293 cells were co-transfected with APP and either empty vector, or active Abl (Abl-PP), or active Src (SrcYF) kinases, and the secretion of APP_{sec} and Aβ were evaluated. Transfected cells have been pulse-labelled with ³⁵S-Met/Cys mix for 30', and chased with cold amino acids for 90'. Both medium and protein extracts have been used in immunoprecipitation experiments for quantitative dosage of radiolabeled APP_{sec} and holo-APP, respectively. While the stability of holo-APP was not affected by co-transfection with either Abl-PP or SrcYF active kinases (Figure 3A), a reduction in the relative amounts of APP_{sec}, normalised to the corresponding holo-APP synthesised during the pulse period was observed in both cases (Figure 3B). This can be interpreted as a decrease of the activity of the α-secretase pathway upon co-transfection of APP with the active kinases.

### Example 3

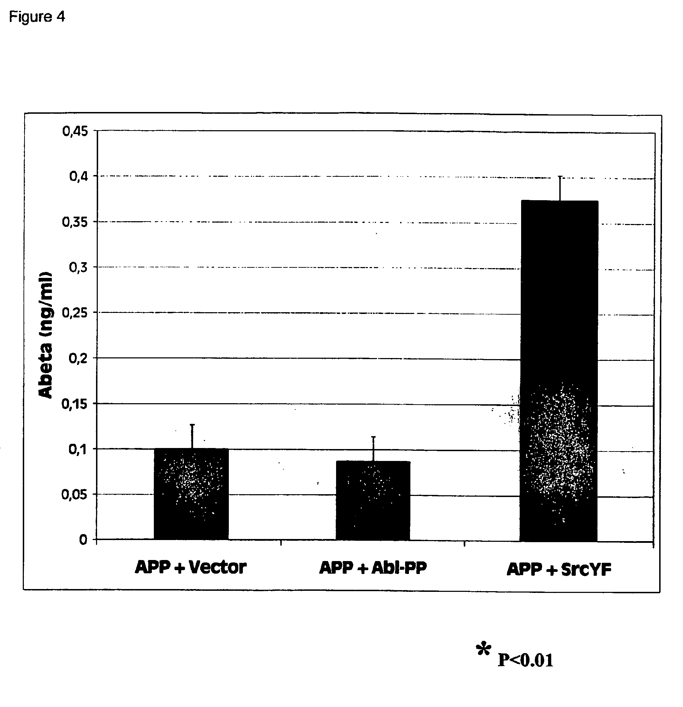

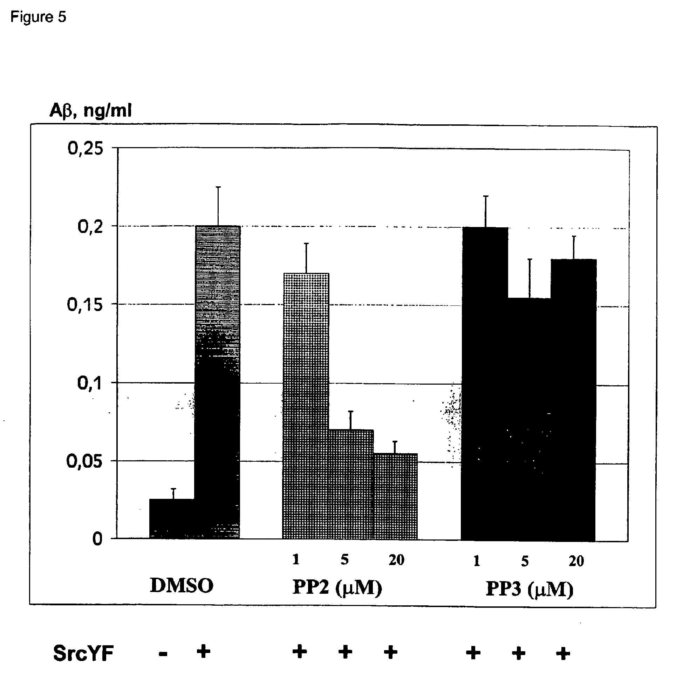

Next, the accumulation of Aβ from cells transfected as above was investigated. The dosage was performed by sandwich ELISA assays on culture medium at 48 hours after transfection (Figure 4). The assay clearly shows that in Src-expressing cells the amount of Aβ secreted is more elevated than in the presence of the control vector, or Abl-PP.
The mechanism by which active Src elicits such a dramatic increase in Aβ secretion is not clear. However, it might depend on phosphorylation of some other proteins, different from APP, since tyrosine phosphorylation of APP by Abl-PP does not result in increased Aβ levels. Accordingly, a similar behaviour is obtained with various APP mutants bearing tyrosine substitutions to either glycine or phenylalanine (data not shown).
Anyway, the rise in Aβ levels does depend on the tyrosine kinase activity of Src, since the accumulation of Aβ is sensitive to treatment by the Src-family specific inhibitor PP2. In fact, as shown in Figure 5, the exposure of transfected cells for 48 hours to increasing concentrations of PP2 results in dose-dependent decrease of secreted Aβ. Either PP3, an inactive analog of PP2, or vehicle alone, do not affect Aβ rise by SrcYF transfection.

Taken together, these observations suggest that exogenous Src activity is responsible for activation of a so far unidentified pathway resulting in increased Aβ secretion.

### REFERENCES

1) T. Russo et al. (1998) FEBS Lett. 434, 1-7.
2) N. Zambrano et al. (1997) J. Biol. Chem. 272, 6399-6405.
3) Sabo S. et al. (1999) J. Biol. Chem. 274, 7952-7957.
4) Borg J.P. et al. (1998) J. Biol. Chem. 273, 14761-14766.
5) lijima, K. et al. (2000) J. Neurochem. 75, 1085-1091.
6) Suzuki, T. et al. (1994) EMBO J. 13, 1114-1122.
7) Oishi, M. et al. (1997) Mol. Med. 3, 111-123.
8) Aplin, E. E. et al. (1996) J. Neurochem. 67, 699-707.
9) Standen, C. L. et al. (2001) J. Neurochem. 76, 316-320.
10) Ando, K. et al. (1999) J. Neurosci. 19, 4421-4427.
11) Ando K. et al. (2001) J Biol Chem.;276, 40353-40361.
12) Zambrano N. et al. (2001) J Biol Chem 276, 19787-19792.
13) Hanke, J. H. et al. (1996) J. Biol. Chem. 271, 695-701.

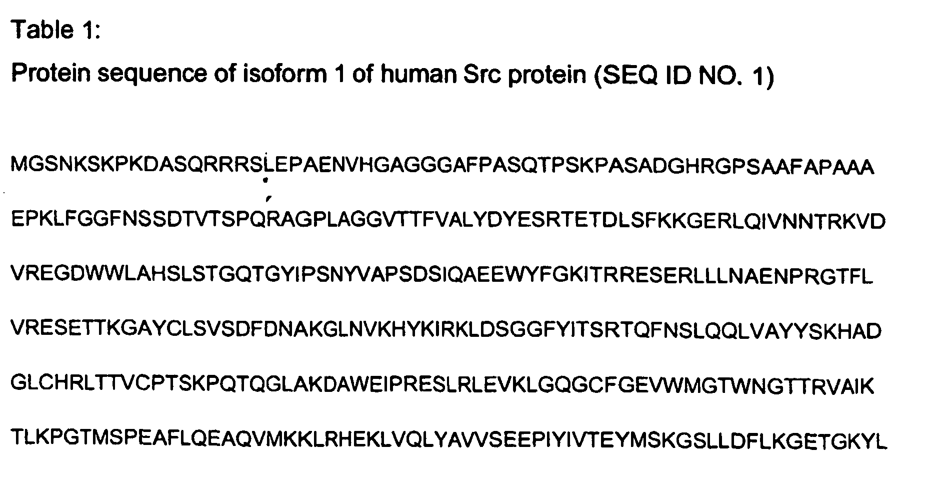

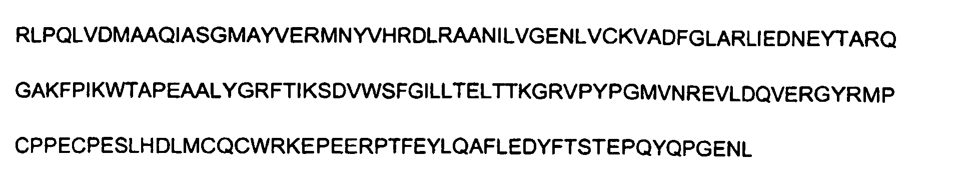

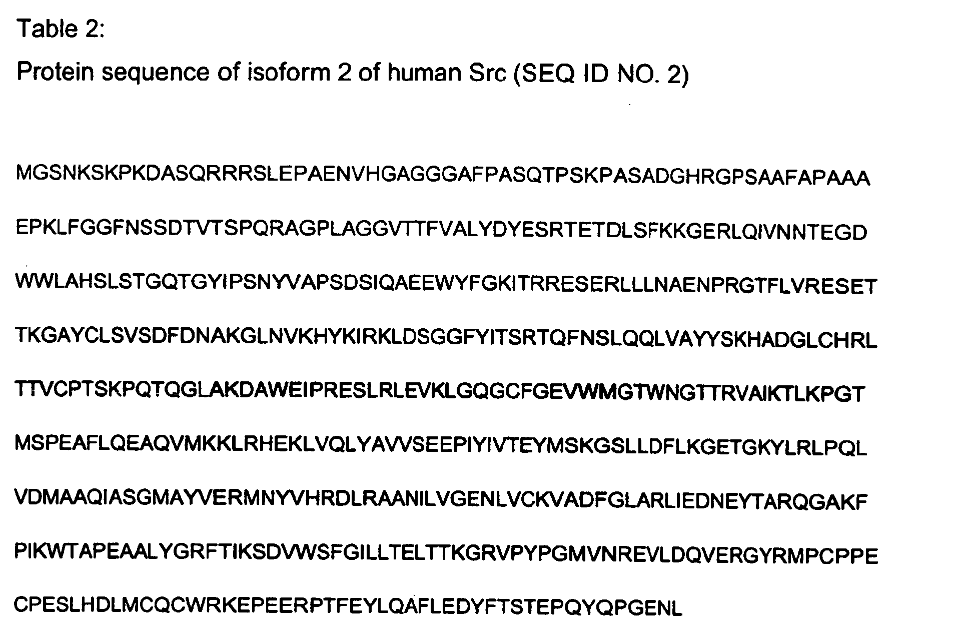

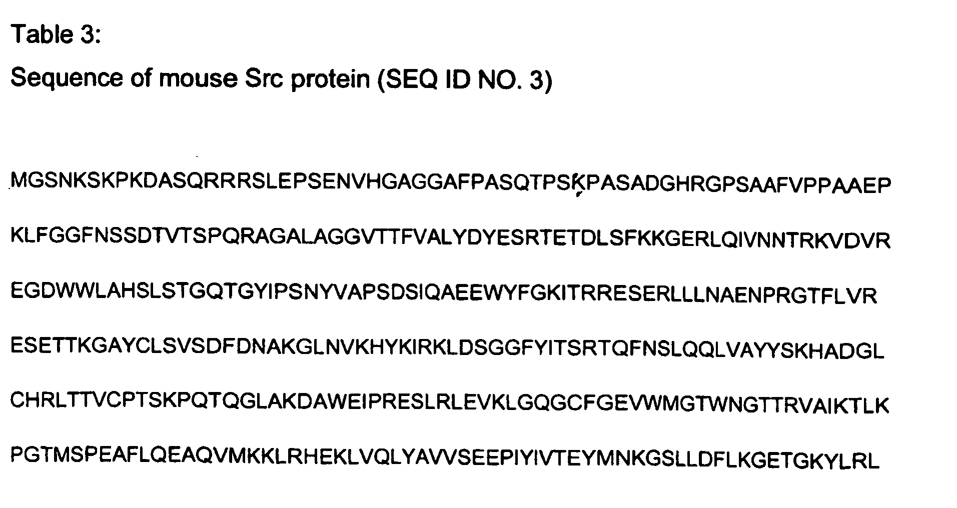

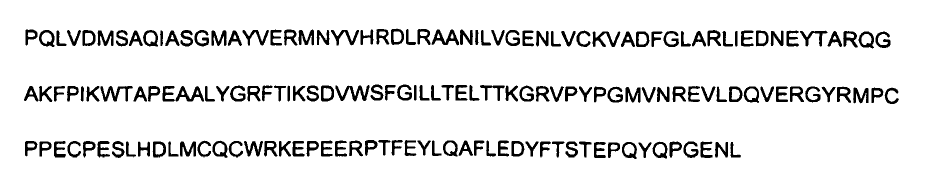

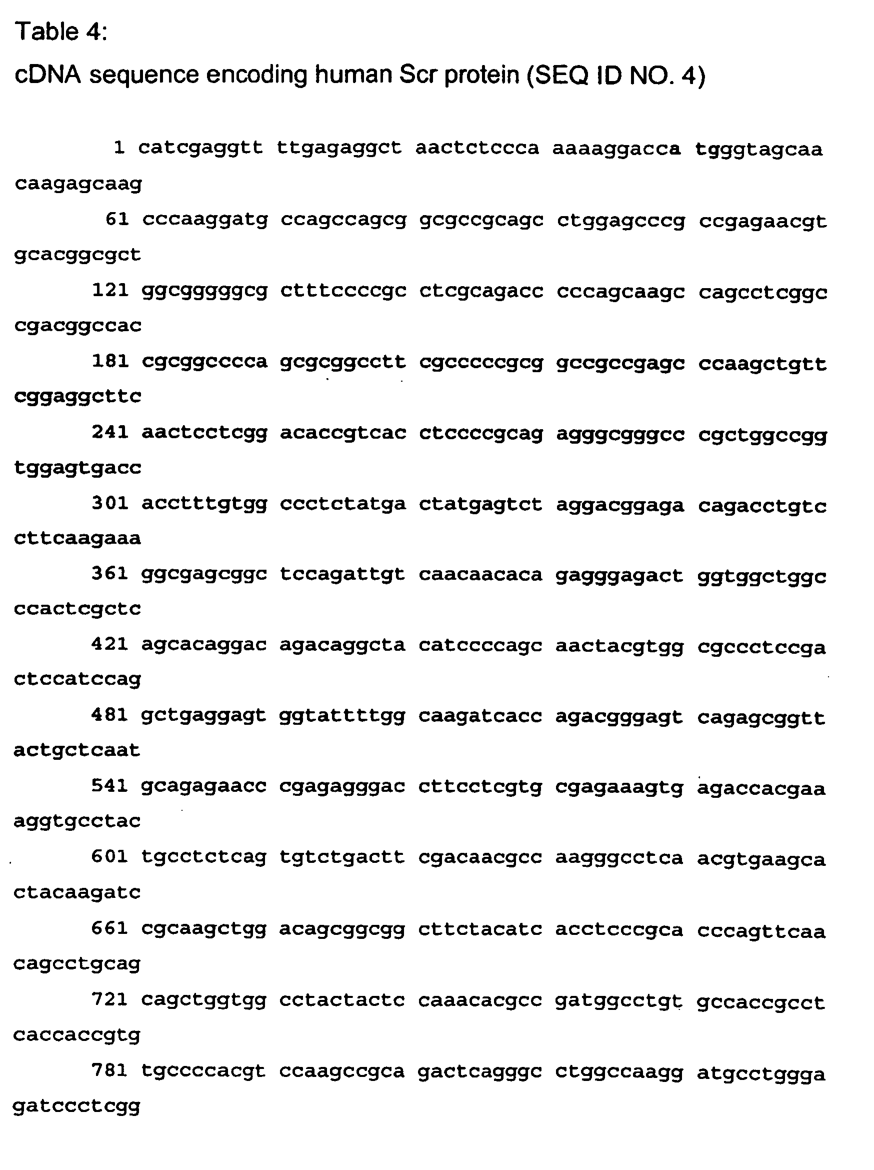

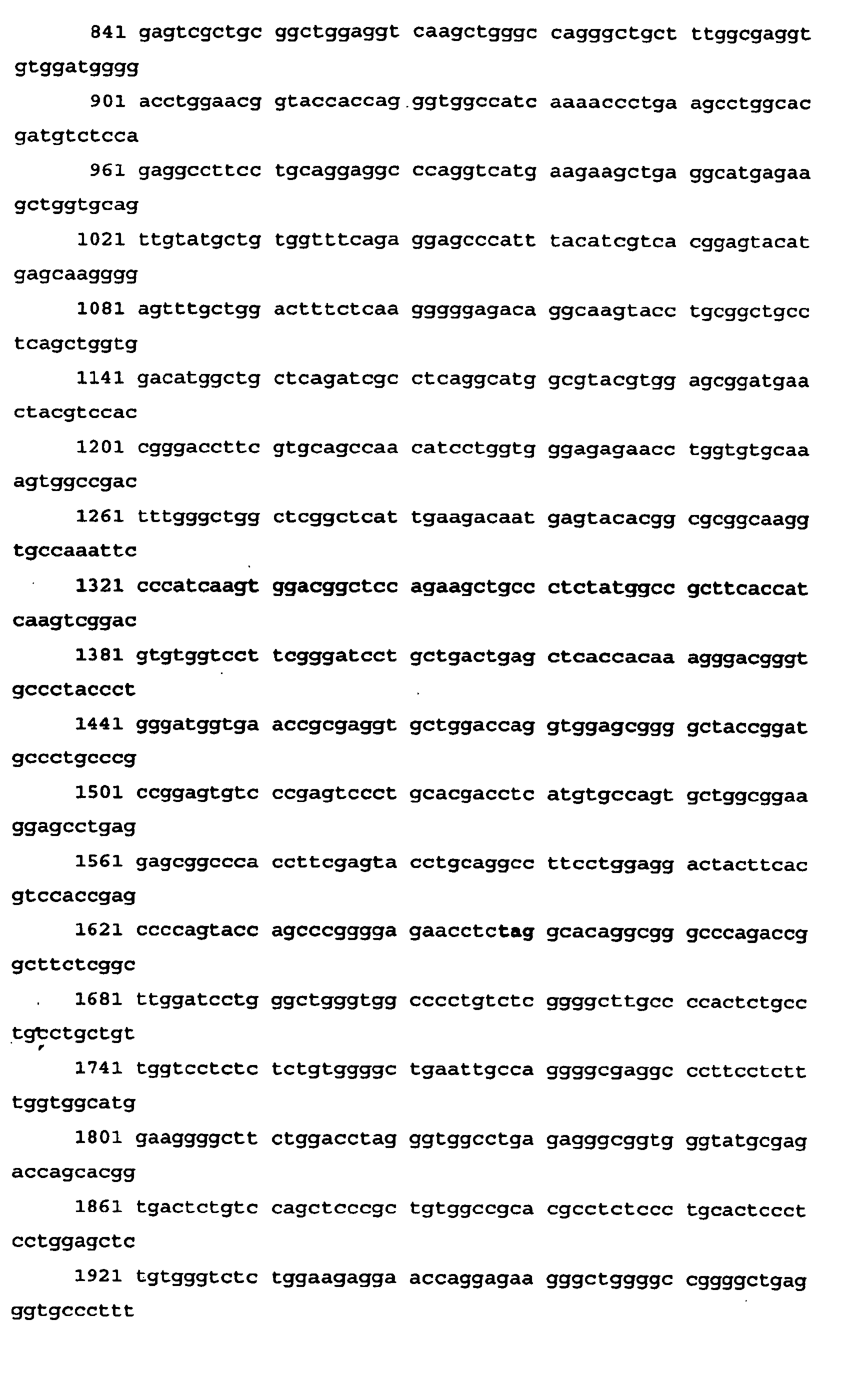

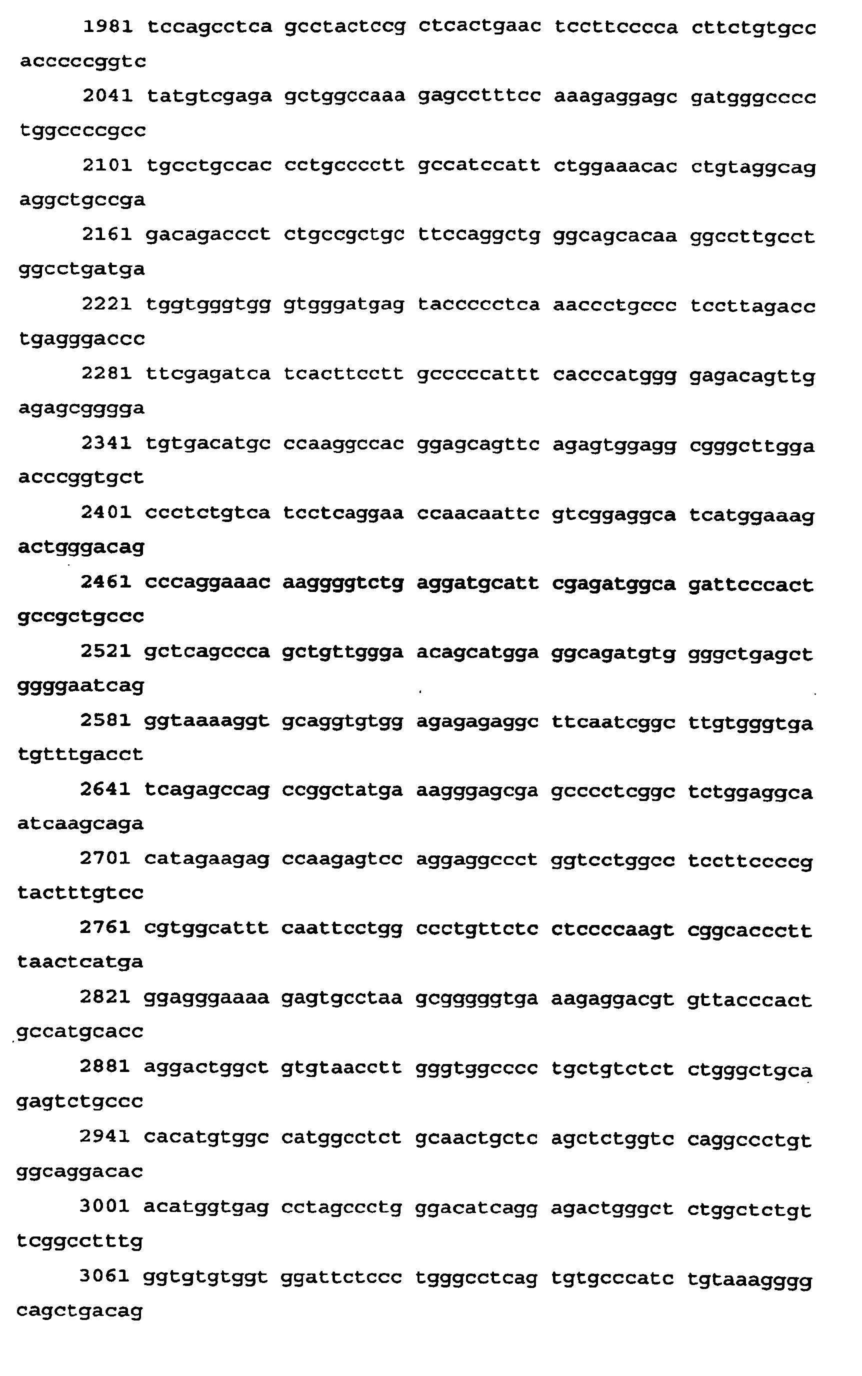

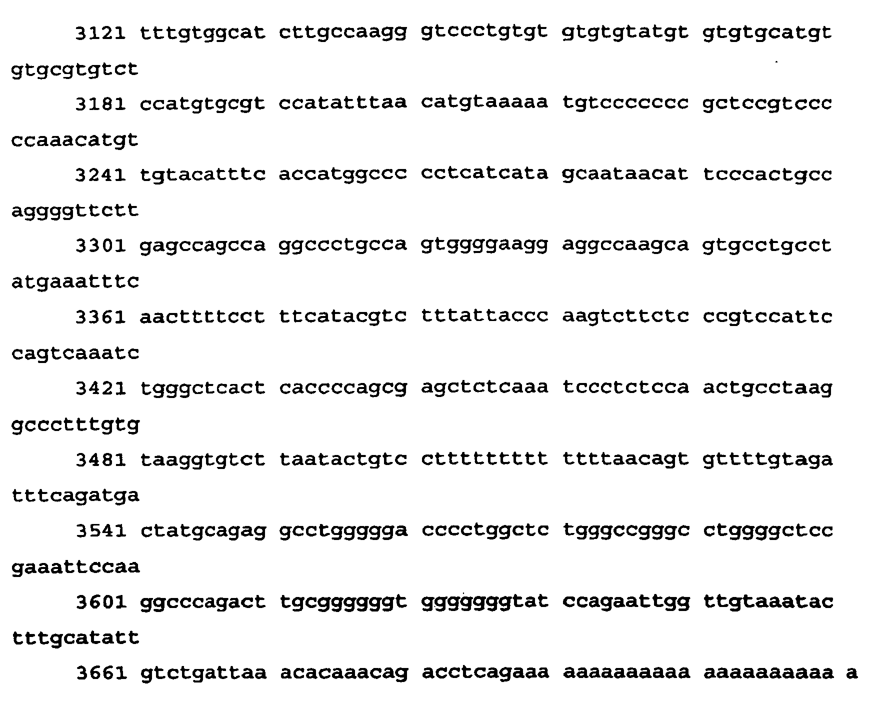

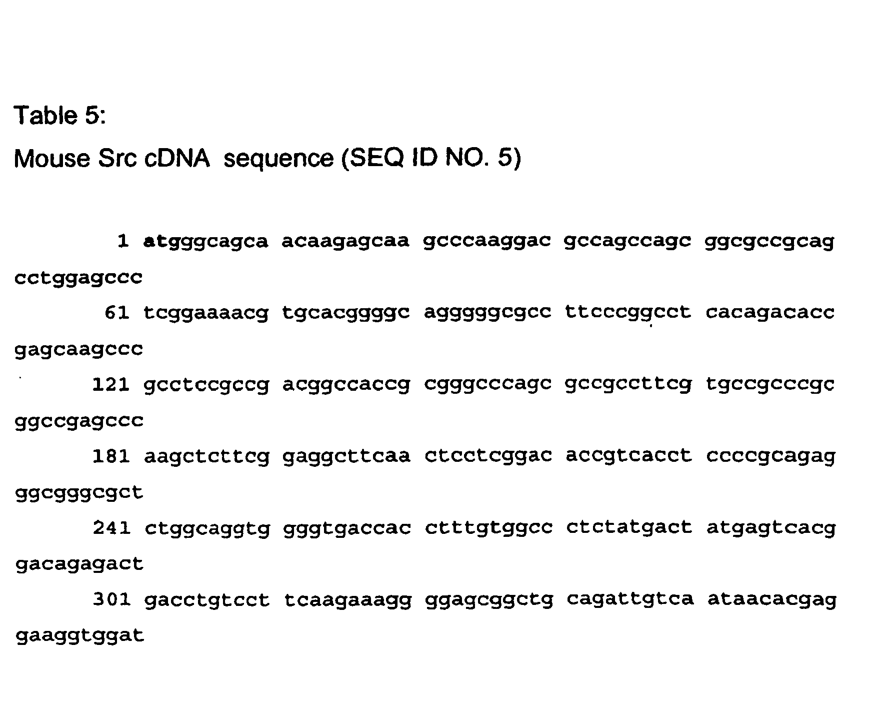

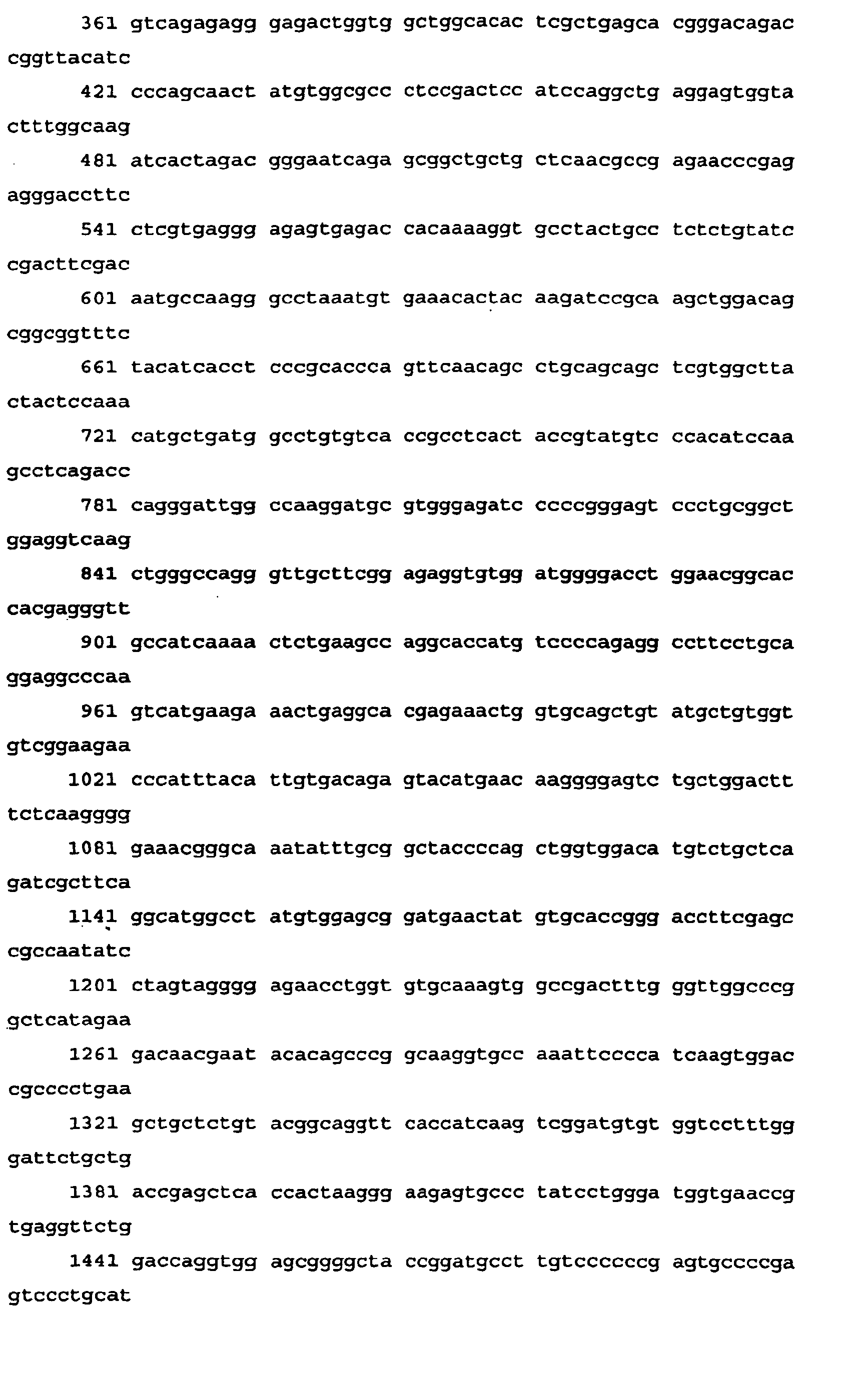

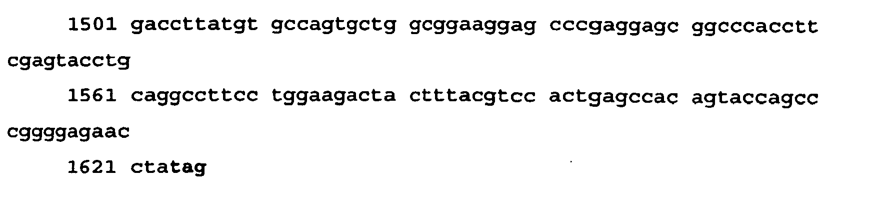

## Claims

1. Method for identifying a therapeutic compound for the treatment of Alzheimer's disease comprising the steps
a) providing a Src protein and
b) determining the inhibitory effect of a compound on the Src kinase activity.

2. Method of identifying a therapeutic compound for the treatment of Alzheimer' s disease comprising the steps
a) providing a sequence which regulates Src expression and
b) determining if a compound inhibits the expression of Src protein.

3. Method according to claim 1 or 2, wherein Src is human Src or mouse Src.

4. Method according to one of the forgoing claims, wherein Src has the sequence SEQ ID NO. 1, SEQ ID NO. 2 or SEQ ID NO. 3.

5. Method according to claim 1, wherein Src is expressed in a mammalian cell.

6. Method according to claim 2; wherein the regulating sequence is the Src promotor.

7. Use of PP2 (4-amino-5-(4-chlorophenyl)-7-(t-butyl)pyrazolo[3,4-d]pyrimidine) for the preparation of a pharmaceutical for the treatment of Alzheimer's disease.

## Patentansprüche

1. Verfahren zur Identifizierung einer therapeutischen Verbindung zur Behandlung der Alzheimer-Krankheit, bei dem man die folgenden Schritte durchführt:
a) Bereitstellen eines Src-Proteins und
b) Bestimmen des Hemmeffekts einer Verbindung auf die Src-Kinase-Aktivität.

2. Verfahren zur Identifizierung einer therapeutischen Verbindung zur Behandlung der Alzheimer-Krankheit, bei dem man die folgenden Schritte durchführt:
a) Bereitstellen einer Sequenz, die die Src-Expression reguliert, und
b) Bestimmen, ob eine Verbindung die Expression von Src-Protein hemmt.

3. Verfahren nach Anspruch 1 oder 2, wobei es sich bei Src um menschliches Src oder Maus-Src handelt.

4. Verfahren nach einem der vorhergehenden Ansprüche, wobei Src die Sequenz SEQ ID NO. 1, SEQ ID NO. 2 oder SEQ ID NO. 3 aufweist.

5. Verfahren nach Anspruch 1, wobei Src in einer Säugerzelle exprimiert wird.

6. Verfahren nach Anspruch 2, wobei es sich bei der regulierenden Sequenz um den Src-Promotor handelt.

7. Verwendung von PP2 (4-Amino-5-(4-chlorphenyl)-7-(t-butyl) pyrazolo[3,4-d]pyrimidin) zur Herstellung eines Medikaments zur Behandlung der Alzheimer-Krankheit.

## Revendications

1. Méthode d'identification d'un composé thérapeutique destiné au traitement de la maladie d'Alzheimer, comprenant les étapes consistant à :
a) fournir une protéine Src, et
b) déterminer l'effet inhibiteur d'un composé sur l'activité kinase de Src.

2. Méthode d'identification d'un composé thérapeutique destiné au traitement de la maladie d'Alzheimer, comprenant les étapes consistant à :
a) fournir une séquence qui régule l'expression de Src, et
b) déterminer si un composé inhibe l'expression de la protéine Src.

3. Méthode selon la revendication 1 ou 2, **caractérisée en ce que** la protéine Src est la protéine Src humaine ou la protéine Src de souris.

4. Méthode selon l'une des revendications précédentes, **caractérisée en ce que** la protéine Src a la séquence SEQ ID NO 1, SEQ ID NO 2 ou SEQ ID NO 3.

5. Méthode selon la revendication 1, **caractérisée en ce que** la protéine Src est exprimée dans une cellule de mammifère.

6. Méthode selon la revendication 2, **caractérisée en ce que** la séquence de régulation est le promoteur de Src.

7. Utilisation de PP2 (4-amino-5-(4-chlorophényl)-7-(t-butyl) pyrazolo [3,4-d] pyrimidine) pour la préparation d'un produit pharmaceutique destiné au traitement de la maladie d'Alzheimer.


### SEQUENCE LISTING

<110> AVENTIS PHARMA S.A.
<120> Inhibitors of SRC kinase for use in Alzheimer's disease
<130> FRAV2002/0030
<160> 5
<170> PatentIn version 3.1
<210> 1
   <211> 542
   <212> PRT
   <213> Homo sapiens
<400> 1
   Met Gly Ser Asn Lys Ser Lys Pro Lys Asp Ala Ser Gln Arg Arg Arg
   1 5 10 15
   Ser Leu Glu Pro Ala Glu Asn Val His Gly Ala Gly Gly Gly Ala Phe
   20 25 30
   Pro Ala Ser Gln Thr Pro Ser Lys Pro Ala Ser Ala Asp Gly His Arg
   35 40 45
   Gly Pro Ser Ala Ala Phe Ala Pro Ala Ala Ala Glu Pro Lys Leu Phe
   50 55 60
   Gly Gly Phe Asn Ser Ser Asp Thr Val Thr Ser Pro Gln Arg Ala Gly
   65 70 75 80
   Pro Leu Ala Gly Gly Val Thr Thr Phe Val Ala Leu Tyr Asp Tyr Glu
   85 90 95
   Ser Arg Thr Glu Thr Asp Leu Ser Phe Lys Lys Gly Glu Arg Leu Gln
   100 105 110
   Ile Val Asn Asn Thr Arg Lys Val Asp Val Arg Glu Gly Asp Trp Trp
   115 120 125
   Leu Ala His Ser Leu Ser Thr Gly Gln Thr Gly Tyr Ile Pro Ser Asn
   130 135 140
   Tyr Val Ala Pro Ser Asp Ser Ile Gln Ala Glu Glu Trp Tyr Phe Gly
   145 150 155 160
   Lys Ile Thr Arg Arg Glu Ser Glu Arg Leu Leu Leu Asn Ala Glu Asn
   165 170 175
   Pro Arg Gly Thr Phe Leu Val Arg Glu Ser Glu Thr Thr Lys Gly Ala
   180 185 190
   Tyr Cys Leu Ser Val Ser Asp Phe Asp Asn Ala Lys Gly Leu Asn Val
   195 200 205
   Lys His Tyr Lys Ile Arg Lys Leu Asp Ser Gly Gly Phe Tyr Ile Thr
   210 215 220
   Ser Arg Thr Gln Phe Asn Ser Leu Gln Gln Leu Val Ala Tyr Tyr Ser
   225 230 235 240
   Lys His Ala Asp Gly Leu Cys His Arg Leu Thr Thr Val Cys Pro Thr
   245 250 255
   Ser Lys Pro Gln Thr Gln Gly Leu Ala Lys Asp Ala Trp Glu Ile Pro
   260 265 270
   Arg Glu Ser Leu Arg Leu Glu Val Lys Leu Gly Gln Gly Cys Phe Gly
   275 280 285
   Glu Val Trp Met Gly Thr Trp Asn Gly Thr Thr Arg Val Ala Ile Lys
   290 295 300
   Thr Leu Lys Pro Gly Thr Met Ser Pro Glu Ala Phe Leu Gln Glu Ala
   305 310 315 320
   Gln Val Met Lys Lys Leu Arg His Glu Lys Leu Val Gln Leu Tyr Ala
   325 330 335
   Val Val Ser Glu Glu Pro Ile Tyr Ile Val Thr Glu Tyr Met Ser Lys
   340 345 350
   Gly Ser Leu Leu Asp Phe Leu Lys Gly Glu Thr Gly Lys Tyr Leu Arg
   355 360 365
   Leu Pro Gln Leu Val Asp Met Ala Ala Gln Ile Ala Ser Gly Met Ala
   370 375 380
   Tyr Val Glu Arg Met Asn Tyr Val His Arg Asp Leu Arg Ala Ala Asn
   385 390 395 400
   Ile Leu Val Gly Glu Asn Leu Val Cys Lys Val Ala Asp Phe Gly Leu
   405 410 415
   Ala Arg Leu Ile Glu Asp Asn Glu Tyr Thr Ala Arg Gln Gly Ala Lys
   420 425 430
   Phe Pro Ile Lys Trp Thr Ala Pro Glu Ala Ala Leu Tyr Gly Arg Phe
   435 440 445
   Thr Ile Lys Ser Asp Val Trp Ser Phe Gly Ile Leu Leu Thr Glu Leu
   450 455 460
   Thr Thr Lys Gly Arg Val Pro Tyr Pro Gly Met Val Asn Arg Glu Val
   465 470 475 480
   Leu Asp Gln Val Glu Arg Gly Tyr Arg Met Pro Cys Pro Pro Glu Cys
   485 490 495
   Pro Glu Ser Leu His Asp Leu Met Cys Gln Cys Trp Arg Lys Glu Pro
   500 505 510
   Glu Glu Arg Pro Thr Phe Glu Tyr Leu Gln Ala Phe Leu Glu Asp Tyr
   515 520 525
   Phe Thr Ser Thr Glu Pro Gln Tyr Gln Pro Gly Glu Asn Leu
   530 535 540
<210> 2
   <211> 536
   <212> PRT
   <213> Homo sapiens
<400> 2
<210> 3
   <211> 541
   <212> PRT
   <213> Murinae gen. sp.
<400> 3
   Met Gly Ser Asn Lys Ser Lys Pro Lys Asp Ala Ser Gln Arg Arg Arg
   1 5 10 15
   Ser Leu Glu Pro Ser Glu Asn Val His Gly Ala Gly Gly Ala Phe Pro
   20 25 30
   Ala Ser Gln Thr Pro Ser Lys Pro Ala Ser Ala Asp Gly His Arg Gly
   35 40 45
   Pro Ser Ala Ala Phe Val Pro Pro Ala Ala Glu Pro Lys Leu Phe Gly
   50 55 60
   Gly Phe Asn Ser Ser Asp Thr Val Thr Ser Pro Gln Arg Ala Gly Ala
   65 70 75 80
   Leu Ala Gly Gly Val Thr Thr Phe Val Ala Leu Tyr Asp Tyr Glu Ser
   85 90 95
   Arg Thr Glu Thr Asp Leu Ser Phe Lys Lys Gly Glu Arg Leu Gln Ile
   100 105 110
   Val Asn Asn Thr Arg Lys Val Asp Val Arg Glu Gly Asp Trp Trp Leu
   115 120 125
   Ala His Ser Leu Ser Thr Gly Gln Thr Gly Tyr Ile Pro Ser Asn Tyr
   130 135 140
   Val Ala Pro Ser Asp Ser Ile Gln Ala Glu Glu Trp Tyr Phe Gly Lys
   145 150 155 160
   Ile Thr Arg Arg Glu Ser Glu Arg Leu Leu Leu Asn Ala Glu Asn Pro
   165 170 175
   Arg Gly Thr Phe Leu Val Arg Glu Ser Glu Thr Thr Lys Gly Ala Tyr
   180 185 190
   Cys Leu Ser Val Ser Asp Phe Asp Asn Ala Lys Gly Leu Asn Val Lys
   195 200 205
   His Tyr Lys Ile Arg Lys Leu Asp Ser Gly Gly Phe Tyr Ile Thr Ser
   210 215 220
   Arg Thr Gln Phe Asn Ser Leu Gln Gln Leu Val Ala Tyr Tyr Ser Lys
   225 230 235 240
   His Ala Asp Gly Leu Cys His Arg Leu Thr Thr Val Cys Pro Thr Ser
   245 250 255
   Lys Pro Gln Thr Gln Gly Leu Ala Lys Asp Ala Trp Glu Ile Pro Arg
   260 265 270
   Glu Ser Leu Arg Leu Glu Val Lys Leu Gly Gln Gly Cys Phe Gly Glu
   275 280 285
   Val Trp Met Gly Thr Trp Asn Gly Thr Thr Arg Val Ala Ile Lys Thr
   290 295 300
   Leu Lys Pro Gly Thr Met Ser Pro Glu Ala Phe Leu Gln Glu Ala Gln
   305                 310                 315                 320
   Val Met Lys Lys Leu Arg His Glu Lys Leu Val Gln Leu Tyr Ala Val
                   325                 330                 335
   Val Ser Glu Glu Pro Ile Tyr Ile Val Thr Glu Tyr Met Asn Lys Gly
               340                 345                 350
   Ser Leu Leu Asp Phe Leu Lys Gly Glu Thr Gly Lys Tyr Leu Arg Leu
           355                 360                 365
   Pro Gln Leu Val Asp Met Ser Ala Gln Ile Ala Ser Gly Met Ala Tyr
       370                 375                 380
   Val Glu Arg Met Asn Tyr Val His Arg Asp Leu Arg Ala Ala Asn Ile
   385                 390                 395                 400
   Leu Val Gly Glu Asn Leu Val Cys Lys Val Ala Asp Phe Gly Leu Ala
                   405                 410                 415
   Arg Leu Ile Glu Asp Asn Glu Tyr Thr Ala Arg Gln Gly Ala Lys Phe
               420                 425                 430
   Pro Ile Lys Trp Thr Ala Pro Glu Ala Ala Leu Tyr Gly Arg Phe Thr
           435                 440                 445
   Ile Lys Ser Asp Val Trp Ser Phe Gly Ile Leu Leu Thr Glu Leu Thr
       450                 455                 460
   Thr Lys Gly Arg Val Pro Tyr Pro Gly Met Val Asn Arg Glu Val Leu
   465                 470                 475                 480
   Asp Gln Val Glu Arg Gly Tyr Arg Met Pro Cys Pro Pro Glu Cys Pro
                   485                 490                 495
   Glu Ser Leu His Asp Leu Met Cys Gln Cys Trp Arg Lys Glu Pro Glu
               500                 505                 510
   Glu Arg Pro Thr Phe Glu Tyr Leu Gln Ala Phe Leu Glu Asp Tyr Phe
           515                 520                 525
   Thr Ser Thr Glu Pro Gln Tyr Gln Pro Gly Glu Asn Leu
       530                 535                 540
<210> 4
   <211> 3711
   <212> DNA
   <213> Homo sapiens
<400> 4
   catcgaggtt ttgagaggct aactctccca aaaaggacca tgggtagcaa caagagcaag 60
   cccaaggatg ccagccagcg gcgccgcagc ctggagcccg ccgagaacgt gcacggcgct 120
   ggcgggggcg ctttccccgc ctcgcagacc cccagcaagc cagcctcggc cgacggccac 180
   cgcggcccca gcgcggcctt cgcccccgcg gccgccgagc ccaagctgtt cggaggcttc 240
   aactcctcgg acaccgtcac ctccccgcag agggcgggcc cgctggccgg tggagtgacc 300
   acctttgtgg ccctctatga ctatgagtct aggacggaga cagacctgtc cttcaagaaa 360
   ggcgagcggc tccagattgt caacaacaca gagggagact ggtggctggc ccactcgctc 420
   agcacaggac agacaggcta catccccagc aactacgtgg cgccctccga ctccatccag 480
   gctgaggagt ggtattttgg caagatcacc agacgggagt cagagcggtt actgctcaat 540
   gcagagaacc cgagagggac cttcctcgtg cgagaaagtg agaccacgaa aggtgcctac 600
   tgcctctcag tgtctgactt cgacaacgcc aagggcctca acgtgaagca ctacaagatc 660
   cgcaagctgg acagcggcgg cttctacatc acctcccgca cccagttcaa cagcctgcag 720
   cagctggtgg cctactactc caaacacgcc gatggcctgt gccaccgcct caccaccgtg 780
   tgccccacgt ccaagccgca gactcagggc ctggccaagg atgcctggga gatccctcgg 840
   gagtcgctgc ggctggaggt caagctgggc cagggctgct ttggcgaggt gtggatgggg 900
   acctggaacg gtaccaccag ggtggccatc aaaaccctga agcctggcac gatgtctcca 960
   gaggccttcc tgcaggaggc ccaggtcatg aagaagctga ggcatgagaa gctggtgcag 1020
   ttgtatgctg tggtttcaga ggagcccatt tacatcgtca cggagtacat gagcaagggg 1080
   agtttgctgg actttctcaa gggggagaca ggcaagtacc tgcggctgcc tcagctggtg 1140
   gacatggctg ctcagatcgc ctcaggcatg gcgtacgtgg agcggatgaa ctacgtccac 1200
   cgggaccttc gtgcagccaa catcctggtg ggagagaacc tggtgtgcaa agtggccgac 1260
   tttgggctgg ctcggctcat tgaagacaat gagtacacgg cgcggcaagg tgccaaattc 1320
   cccatcaagt ggacggctcc agaagctgcc ctctatggcc gcttcaccat caagtcggac 1380
   gtgtggtcct tcgggatcct gctgactgag ctcaccacaa agggacgggt gccctaccct 1440
   gggatggtga accgcgaggt gctggaccag gtggagcggg gctaccggat gccctgcccg 1500
   ccggagtgtc ccgagtccct gcacgacctc atgtgccagt gctggcggaa ggagcctgag 1560
   gagcggccca ccttcgagta cctgcaggcc ttcctggagg actacttcac gtccaccgag 1620
   ccccagtacc agcccgggga gaacctctag gcacaggcgg gcccagaccg gcttctcggc 1680
   ttggatcctg ggctgggtgg cccctgtctc ggggcttgcc ccactctgcc tgcctgctgt 1740
   tggtcctctc tctgtggggc tgaattgcca ggggcgaggc ccttcctctt tggtggcatg 1800
   gaaggggctt ctggacctag ggtggcctga gagggcggtg ggtatgcgag accagcacgg 1860
   tgactctgtc cagctcccgc tgtggccgca cgcctctccc tgcactccct cctggagctc 1920
   tgtgggtctc tggaagagga accaggagaa gggctggggc cggggctgag ggtgcccttt 1980
   tccagcctca gcctactccg ctcactgaac tccttcccca cttctgtgcc acccccggtc   2040
   tatgtcgaga gctggccaaa gagcctttcc aaagaggagc gatgggcccc tggccccgcc   2100
   tgcctgccac cctgcccctt gccatccatt ctggaaacac ctgtaggcag aggctgccga   2160
   gacagaccct ctgccgctgc ttccaggctg ggcagcacaa ggccttgcct ggcctgatga   2220
   tggtgggtgg gtgggatgag taccccctca aaccctgccc tccttagacc tgagggaccc   2280
   ttcgagatca tcacttcctt gcccccattt cacccatggg gagacagttg agagcgggga   2340
   tgtgacatgc ccaaggccac ggagcagttc agagtggagg cgggcttgga acccggtgct   2400
   ccctctgtca tcctcaggaa ccaacaattc gtcggaggca tcatggaaag actgggacag   2460
   cccaggaaac aaggggtctg aggatgcatt cgagatggca gattcccact gccgctgccc   2520
   gctcagccca gctgttggga acagcatgga ggcagatgtg gggctgagct ggggaatcag   2580
   ggtaaaaggt gcaggtgtgg agagagaggc ttcaatcggc ttgtgggtga tgtttgacct   2640
   tcagagccag ccggctatga aagggagcga gcccctcggc tctggaggca atcaagcaga   2700
   catagaagag ccaagagtcc aggaggccct ggtcctggcc tccttccccg tactttgtcc   2760
   cgtggcattt caattcctgg ccctgttctc ctccccaagt cggcaccctt taactcatga   2820
   ggagggaaaa gagtgcctaa gcgggggtga aagaggacgt gttacccact gccatgcacc   2880
   aggactggct gtgtaacctt gggtggcccc tgctgtctct ctgggctgca gagtctgccc   2940
   cacatgtggc catggcctct gcaactgctc agctctggtc caggccctgt ggcaggacac   3000
   acatggtgag cctagccctg ggacatcagg agactgggct ctggctctgt tcggcctttg   3060
   ggtgtgtggt ggattctccc tgggcctcag tgtgcccatc tgtaaagggg cagctgacag   3120
   tttgtggcat cttgccaagg gtccctgtgt gtgtgtatgt gtgtgcatgt gtgcgtgtct   3180
   ccatgtgcgt ccatatttaa catgtaaaaa tgtccccccc gctccgtccc ccaaacatgt   3240
   tgtacatttc accatggccc cctcatcata gcaataacat tcccactgcc aggggttctt   3300
   gagccagcca ggccctgcca gtggggaagg aggccaagca gtgcctgcct atgaaatttc   3360
   aacttttcct ttcatacgtc tttattaccc aagtcttctc ccgtccattc cagtcaaatc   3420
   tgggctcact caccccagcg agctctcaaa tccctctcca actgcctaag gccctttgtg   3480
   taaggtgtct taatactgtc cttttttttt ttttaacagt gttttgtaga tttcagatga   3540
   ctatgcagag gcctggggga cccctggctc tgggccgggc ctggggctcc gaaattccaa   3600
   ggcccagact tgcggggggt gggggggtat ccagaattgg ttgtaaatac tttgcatatt   3660
   gtctgattaa acacaaacag acctcagaaa aaaaaaaaaa aaaaaaaaaa a            3711
<210> 5
   <211> 1626
   <212> DNA
   <213> Murinae gen. sp.
<400> 5
   atgggcagca acaagagcaa gcccaaggac gccagccagc ggcgccgcag cctggagccc     60
   tcggaaaacg tgcacggggc agggggcgcc ttcccggcct cacagacacc gagcaagccc    120
   gcctccgccg acggccaccg cgggcccagc gccgccttcg tgccgcccgc ggccgagccc    180
   aagctcttcg gaggcttcaa ctcctcggac accgtcacct ccccgcagag ggcgggcgct    240
   ctggcaggtg gggtgaccac ctttgtggcc ctctatgact atgagtcacg gacagagact    300
   gacctgtcct tcaagaaagg ggagcggctg cagattgtca ataacacgag gaaggtggat    360
   gtcagagagg gagactggtg gctggcacac tcgctgagca cgggacagac cggttacatc    420
   cccagcaact atgtggcgcc ctccgactcc atccaggctg aggagtggta ctttggcaag    480
   atcactagac gggaatcaga gcggctgctg ctcaacgccg agaacccgag agggaccttc    540
   ctcgtgaggg agagtgagac cacaaaaggt gcctactgcc tctctgtatc cgacttcgac    600
   aatgccaagg gcctaaatgt gaaacactac aagatccgca agctggacag cggcggtttc    660
   tacatcacct cccgcaccca gttcaacagc ctgcagcagc tcgtggctta ctactccaaa    720
   catgctgatg gcctgtgtca ccgcctcact accgtatgtc ccacatccaa gcctcagacc    780
   cagggattgg ccaaggatgc gtgggagatc ccccgggagt ccctgcggct ggaggtcaag    840
   ctgggccagg gttgcttcgg agaggtgtgg atggggacct ggaacggcac cacgagggtt    900
   gccatcaaaa ctctgaagcc aggcaccatg tccccagagg ccttcctgca ggaggcccaa    960
   gtcatgaaga aactgaggca cgagaaactg gtgcagctgt atgctgtggt gtcggaagaa   1020
   cccatttaca ttgtgacaga gtacatgaac aaggggagtc tgctggactt tctcaagggg   1080
   gaaacgggca aatatttgcg gctaccccag ctggtggaca tgtctgctca gatcgcttca   1140
   ggcatggcct atgtggagcg gatgaactat gtgcaccggg accttcgagc cgccaatatc   1200
   ctagtagggg agaacctggt gtgcaaagtg gccgactttg ggttggcccg gctcatagaa   1260
   gacaacgaat acacagcccg gcaaggtgcc aaattcccca tcaagtggac cgcccctgaa   1320
   gctgctctgt acggcaggtt caccatcaag tcggatgtgt ggtcctttgg gattctgctg   1380
   accgagctca ccactaaggg aagagtgccc tatcctggga tggtgaaccg tgaggttctg   1440
   gaccaggtgg agcggggcta ccggatgcct tgtccccccg agtgccccga gtccctgcat   1500
   gaccttatgt gccagtgctg gcggaaggag cccgaggagc ggcccacctt cgagtacctg   1560
   caggccttcc tggaagacta ctttacgtcc actgagccac agtaccagcc cggggagaac   1620
   ctatag                                                              1626